**Europäisches Patentamt**

**European Patent Office**

**Office européen des brevets**

(19)

(11) Veröffentlichungsnummer : **0 172 838**
**B1**

(12) **EUROPÄISCHE PATENTSCHRIFT**

(45) Veröffentlichungstag der Patentschrift :
25.05.88

(21) Anmeldenummer : 85900627.2

(22) Anmeldetag : 13.02.85

(86) Internationale Anmeldenummer :
PCT/CH 85/00027

(87) Internationale Veröffentlichungsnummer :
WO/8503773 (29.08.85 Gazette 85/19)

(51) Int. Cl.⁴ : **G 01 N 1/10, C 12 M 1/26**

(54) PROBENAHME - VORRICHTUNG.

(30) Priorität : 20.02.84 CH 794/84

(43) Veröffentlichungstag der Anmeldung :
05.03.86 Patentblatt 86/10

(45) Bekanntmachung des Hinweises auf die Patenterteilung : 25.05.88 Patentblatt 88/21

(84) Benannte Vertragsstaaten :
AT BE CH DE FR GB LI LU NL SE

(56) Entgegenhaltungen :
WO-A-83 /033 02
WO-A-84 /005 59
DE-A- 2 614 542
DE-A- 2 907 558
FR-A- 2 374 629
FR-A- 2 486 240

(73) Patentinhaber : Meyer, Pio
Sagenrainstrasse 7
CH-8636 Wald (CH)

(72) Erfinder : Meyer, Pio
Sagenrainstrasse 7
CH-8636 Wald (CH)

(74) Vertreter : Salgo, Reinhold Caspar
Usterstrasse 139
CH-8621 Wetzikon 4 (CH)

**Beschreibung**

Die vorliegende Erfindung bezieht sich auf eine Vorrichtung zur Entnahme von Proben aus beispielsweise Bio-Reaktoren oder Fermentern, Chemie-Reaktoren und Lagertanks und zum Abfüllen dieser Proben in sterile Gefässe.

Die Entnahme von Kulturproben aus einem Fermenter ist immer dann angezeigt, wenn die Prozess-Parameter wie pH, pO2, pCO2, Temperatur, Viskosität und andere, keinen genauen Aufschluss über den Zustand der intrazellulären Parameter der Kultur zulassen. Aus diesem Grunde ist die Forderung nach einer Vorrichtung zur Entnahme solcher Kulturproben — eben einer Probenahme-Vorrichtung — so alt wie die Technik des Fermentierens in abgeschlossenen Behältern. Dass die Probe zudem in ein steriles Gefäss abgefüllt werden soll ist immer dann notwendig, wenn allenfalls im Gefäss vorhandene Keime die Probe verfälschen können. Solche Vorrichtungen sind daher bekannt, beispielsweise aus der CH-PS 629 591.

In dieser den Stand der Technik darstellenden Patentschrift ist eine Vorrichtung beschrieben, die besteht aus einem Entnahme-Rohr, einem Ventil — beispielsweise einem Eckventil — und einer glockenförmigen Halterung für die sterile Flasche. Das Entnahmerohr wird mittels einer Schraubverbindung oder mit einem Flansch in einer Oeffnung des Fermenters befestigt. Vom geöffneten Eckventil strömt die Kulturbrühe in eine erste — längere Nadel, die durch den elastomeren Stopfen der sterilen Flasche hindurchgesteckt ist, in diese Flasche. Die darin enthaltene Luft entweicht über eine zweite — kürzere Nadel, die ebenfalls durch den Stopfen hindurchgesteckt ist, nach aussen. Die Problematik dieser und ähnlicher Vorrichtungen liegt in der Sterilisierbarkeit in dreifacher Hinsicht :

Geht man davon aus, dass das Innere des Entnahmerohrs, des Eckventils und der längeren Nadel zusammen mit dem Fermenter vor dem Ingangsetzen des Fermentationsprozesses sterilisiert wurde, so ist für die erste Probenahme Sterilität immer noch nicht gewährleistet, da das Aeussere des Stopfens der nichtsterilen Laborluft ausgesetzt ist.

Nach dem Entnehmen der ersten Probe und dem Entfernen des Gefässes sind Nadel und Eckventil bis zum Ventilsitz nicht mehr steril und können auch nicht mehr sterilisiert werden. Zudem sitzen im Entnahmerohr mindestens Reste der Kulturbrühe.

Falls im Fermenter pathogene Kulturen gezogen werden, gelangen Keime in's Labor, sobald das Gefäss von der Nadel genommen wird. Weitere bekannte Vorrichtungen arbeiten mit einem Becher, der während der Dampfsterilisation über die Nadel gestülpt und nach Beendigung der Sterilisation wieder entfernt wird. Damit ist immer die Möglichkeit der Kontamination der Probe und, in den Fällen pathogener Kulturen, des Labors gegeben.

Die der vorliegenden Erfindung zugrunde liegende Aufgabe war es, eine Probenahme-Vorrichtung zu schaffen, die sowohl mit dem Fermenter zusammen, als auch in situ sterilisiert werden kann, die nach jeder Probenahme gespült und neu sterilisiert werden kann, die so beschaffen ist, dass das Aeussere des Stopfens des sterilen Aufnahmegefässes für die Kulturprobe ebenfalls mitsterilisiert werden kann ; dass die Vorrichtung gleichermassen verwendbar ist für die Entnahme von Proben aus pathogenen Kulturen und dass diese Proben nie mit der Atmosphäre in Berührung kommen.

Die Lösung dieser Aufgabe zeichnet sich aus durch das Vorhandensein dreier hauptsächlicher Bauteile, nämlich eines hohlen Hauptschaftes, der mit dem Gefäss, aus dem die Probe entnommen werden soll, fest verbunden wird, eines dazu axial beweglichen Entnahmerohres und eines zum Entnahmerohr axial beweglichen Flaschenhalters ; weitere kennzeichnendes Merkmal ist das Vorhandensein je eines Rohres, sowohl am Hauptschaft, als auch am Flaschenhalter, ferner eines stirnseitigen Verschlusses und einer seitlichen Bohrung am Entnahmerohr und die Ausgestaltung des Entnahmerohres als Nadel an der dem dem stirnseitigen Verschluss gegenüberliegenden Ende des Entnahmerohres, dergestalt, dass das Sterilisationsmedium durch das am Hauptschaft befindliche Rohr in den Hauptschaft, dann durch die seitliche Bohrung ins Innere des Entnahmerohres, durch die Nadel ins Innere des Flaschenhalters, und von dort durch das zweite Rohr nach aussen strömen kann. In der Entnahmestellung, die durch Einwirken einer Axialen Kraft auf die Vorrichtung erreicht wird, strömt die zu entnehmende Flüssigkeit oder Brühe durch die, nun ins Innere des Behälters reichende, Bohrung am Entnahmerohr, dann durch dieses und die Nadel, die durch den Pfropfen einer vom Flaschenhalter gehaltenen Probeflasche durch die genannte axiale Kraft hindurchgetreten ist, ins Innere der genannten Probeflasche.

In der beiliegenden Zeichnung ist ein Ausführungsbeispiel der Erfindung näher erläutert.

Es zeigen

Fig. 1 einen Längsschnitt durch die an einen Fermenter angebaute erfindugsgemässe Probenahme-Vorrichtung in Sterilisationsstellung

Fig. 2 das Ausführungsbeispiel gem. Fig. 1 in Arbeitsstellung I.

Fig. 3 dasselbe Ausführungsbeispiel in Arbeitsstellung II.

Fig. 4 ein zweites Ausführungsbeispiel, ebenfalls im Längsschnitt.

Fig. 5 Eine Variante zum Ausführungsbeispiel gemäss Fig. 1.

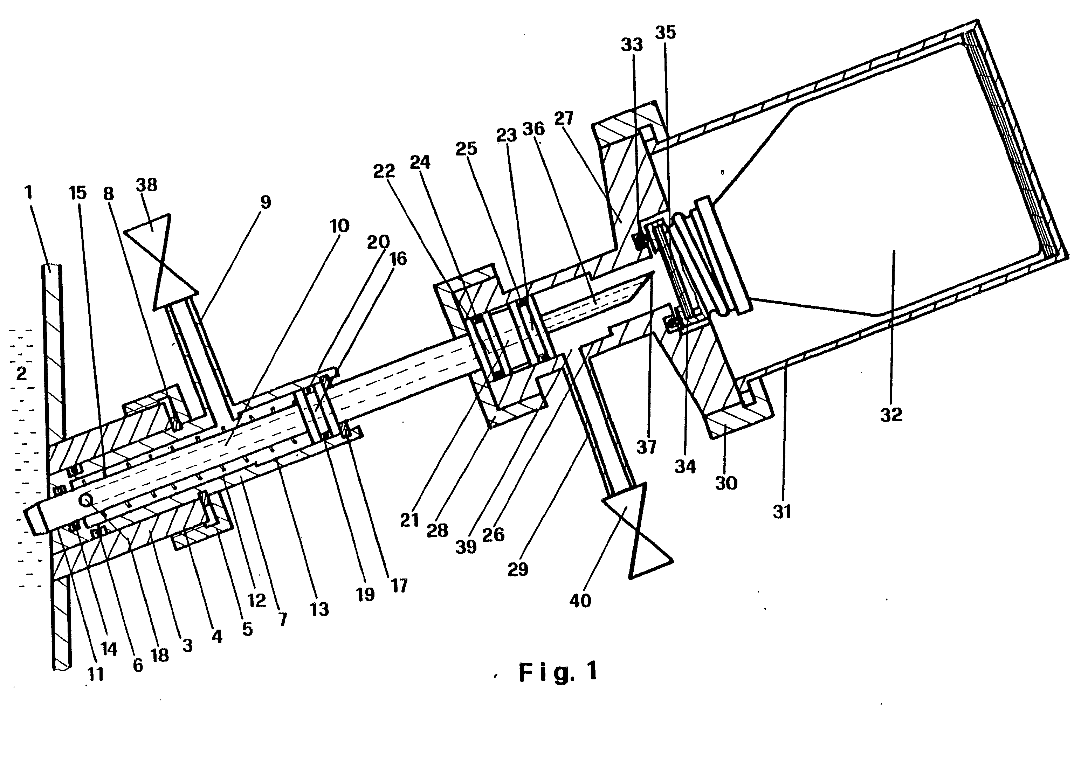

In einem Fermenter, dessen Wandung 1 in Fig. 1 dargestellt ist, ist Kulturbrühe 2 enthalten. In die Wandung 1 ist ein Stutzen, hier ein Normstutzen 3 eingeschweisst, der mit einem Gewinde 4 für die Aufnahme einer Ueberwurfmutter 5 versehen ist.

Eingesteckt in den Normstutzen 3, und mit Hilfe eines O-Rings 6 abgedichtet, ist der hohle Hauptschaft 7 der Porbenahme-Vorrichtung. Die Ueberwurfmutter 5 sichert den Hauptschaft 7 gegen Herausziehen, indem sie gegen einen Ring 8 am Hauptschaft drückt. In den hohlen Hauptschaft 7 mündet ein Rohr 9 mit einem Absperrorgan 38, durch das das Sterilisationsmedium zugeführt wird. Der Hauptschaft 7 ist innen ausgestaltet zur Aufnahme eines beweglichen Entnahmerohres 10. Zu diesem Zwecke weist er drei verschieden weite Bohrungen 11,12,13 auf :

— Die Bohrung 11, die so bemessen ist, dass ein O-Ring 14, der im vorderen Ende des Hauptschaftes 7 eingelassen ist, mit dem Entnahmerohr einen Dichtsitz gegen die Kulturbrühe 2 bildet ;

— die Bohrung 12, die weiter ist als die Bohrung 11 und so weit, dass eine Druckfeder 15, die das Entnahmerohr 10 umgibt, mit genügend Spiel, ohne den Hauptschaft 7 zu berühren, darin bewegt werden kann ;

— die Bohrung 13, wiederum weiter als die Bohrung 12, erlaubt einer Führung 16 eine Längsbewegung, die nach hinten, also vom Fermenter weg, begrenzt ist durch einen weiteren Ring 17.

Das Entnahmerohr 10 ist vorne, also gegen die Kulturbrühe hin, verschlossen. Etwas zurückversetzt trägt es seitlich eine durchgehende Bohrung 18, die den Zugang in sein Inneres freigibt. Die Führung 16 ist fest mit dem Entnahmerohr 10 verbunden und ist zu Dichtzwecken mit einer Nut 19 versehen, in der ein O-Ring 20 liegt. Die Druckfeder 15 ist unter Vorspannung eingesetzt und zieht, sofern nicht äussere Krafteinwirkung dem entgegensteht, das Entnahmerohr 10 in's Innere des Hauptschaftes 7 zurück.

Wiederum fest verbunden mit dem Entnahmerohr 10 ist eine weitere Führung 21. Sie trägt zwei Nuten 22,23 in denen zwei O-Ringe 24,25 liegen. Die O-Ringe 24,25 bilden Dichtsitze mit einer Bohrung 26 in einem Bauteil, der mit Flaschenhalter 27 bezeichnet wird.

Gegen Zurückziehen aus dem Flaschenhalter 27 ist die Führung 21 gesichert durch eine Ueberwurfmutter 28. In die Bohrung 26 mündet ein Rohr 29, das zur Ableitung des Sterilisationsmediums, von Spülflüssigkeit oder Kondensat dient, je nachdem, was durch das Rohr 9 zugeführt wird. Mit Hilfe einer weiteren Ueberwurfmutter 30 ist an dem Flaschenhalter 27 ein Becher 31 befestigt, der eine Flasche 32, die zur Aufnahme der Kulturprobe dienen soll, gegen einen O-Ring 33 presst. Der O-Ring 33 dichtet gegen den Schraubdeckel 34 der Flasche 32, der einen geeignet geformten Pfropfen 35 aus elastomerem Material niederhält. Die Flasche 32 ist durch den Pfopfen 35 dicht verschlossen. Das hintere, also der Flasche 32 zugewandte, Ende des Aufnahmerohrs 10, trägt eine Nadel 36, die schräg angespitzt ist zu einer Spitze 37.

In dieser Stellung der Probenahme-Vorrichtung besteht eine durchgehende Verbindung vom Rohr 9 bis zum Rohr 29 :

Das Sterilisationsmedium, beispielsweise Dampf, strömt ein durch das Rohr 9, umspült das Aeussere des Entnahmerohres 10 mitsamt der Druckfeder 15, gelangt durch die Bohrung 18 ins Innere des Entnahmerohres 10 und verlässt dieses bei der Spitze 37 der Nadel 36. Das Innere des Flaschenhalters 27 mitsamt der durch den O-Ring 33 freigegebenen Partien des Schraubdeckels 34 und des Stopfens 35, begrenzt durch den O-Ring 25, wird ebenfalls vom Dampf durchströmt und damit sterilisiert. Der Dampf mitsamt allfälligem Kondensat verlässt die Vorrichtung durch das Rohr 29. Alle Bauteile, die später mit der Kulturprobe in Kontakt kommen, sind nun steril.

Nach Beendigung des Sterilisationsvorganges wird das Absperrorgan 38 geschlossen, ebenso ein weiteres Absperrorgan 40 am Rohr 29 und auf den Boden des Bechers 31 eine zum Entnahmerohr koaxiale Kraft aufgebracht. Diese schiebt die Flasche gegen die Spitze 37, die nun in den Stopfen 35 eindringen kann. Zugleich schiebt sich der O-Ring 25 über die Mündung 39 des Rohres 29, die nun zwischen die O-Ringe 24 und 25 zu liegen kommt ; gleichzeitig ist die Spitze 37 der Nadel 36 durch den Stopfen 35 eingedrungen und die Führung 21 am Ende der Bohrung 26 angekommen.

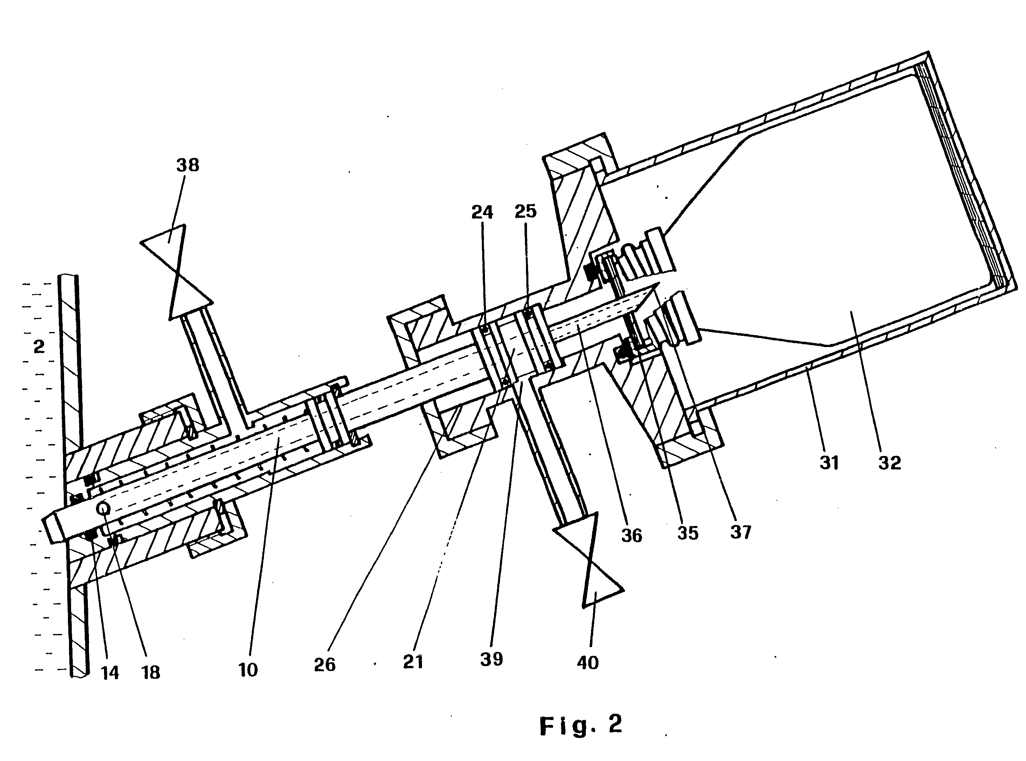

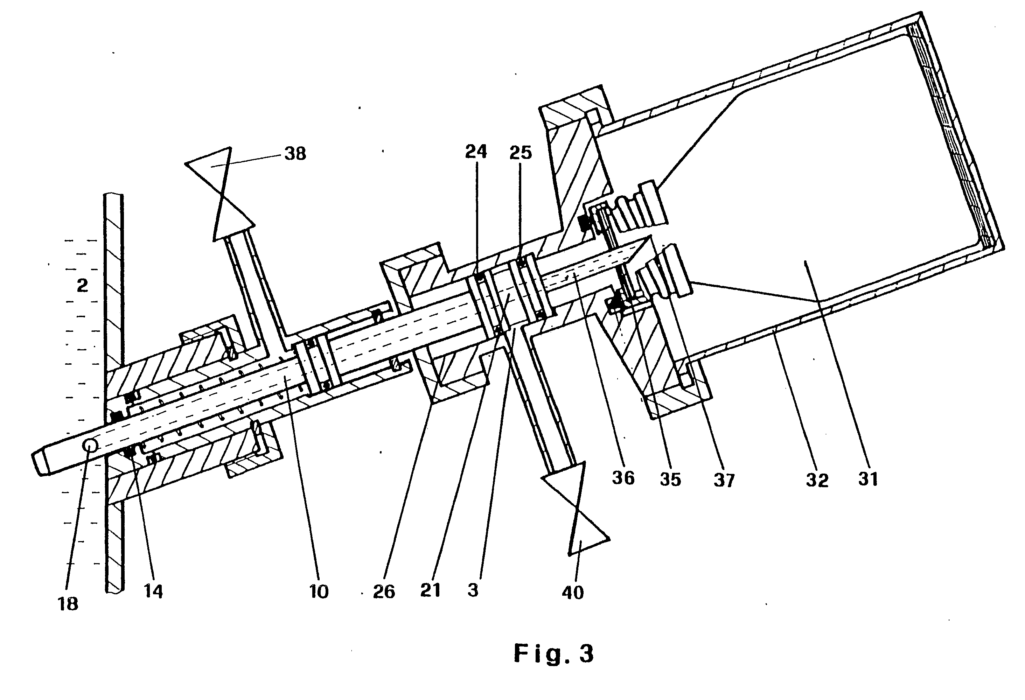

Diese Situation ist in Fig. 2 als Arbeitsstellung I dargestellt. Sie ist nur Durchgangsphase für die in Fig. 3 dargestellte Arbeitsstellung II, zugleich aber auch Durchgangsphase auf dem Weg von der Arbeitsstellung II zurück zur Sterilisationsstellung gemäss Fig. 1, in der dann die Ueberwurfmutter 30 gelöst und die Flasche 32 dem Becher 31 entnommen werden kann. Dieser Operation wird eine Spül- und bei pathogenen Kulturen, eine Sterilisationsphase vorausgehen.

Wird nun die genannte Kraft auf den Boden des Bechers 31 weiter verstärkt, so schiebt sich das Entnahmerohr durch den O-Ring 14 in die im Fermenter enthaltene Kulturbrühe 2. Diese tritt, sobald die Bohrung 18 durch den O-Ring 14 freigegeben ist, in's Innere des Entnahmerohres 10 und durch die Nadel 36 in die Flasche 32. Trotzdem bei dem in Fig. 1 bis 3 dargestellten Ausführungsbeispiel keine Entlüftung der Flasche 32 vorgesehen ist, kann die Kulturbrühe 2 in die Flasche 32 eindringen, da im Innern des Fermenters ein kleiner Ueberdruck herrscht oder leicht vorübergehend hergestellt werden kann, und im Innern der Flasche 32 im allgemeinen — von der Sterilisation her — ein Unterdruck bezüglich der Atmosphäre. Dieser Unterdruck wird beim Eindringen der Nadel 36 in die Flasche 32 nur wenig abgebaut, da das Volumen der ganzen Probenahme-Vorrichtung wesentlich kleiner ist als jenes der Flasche 32.

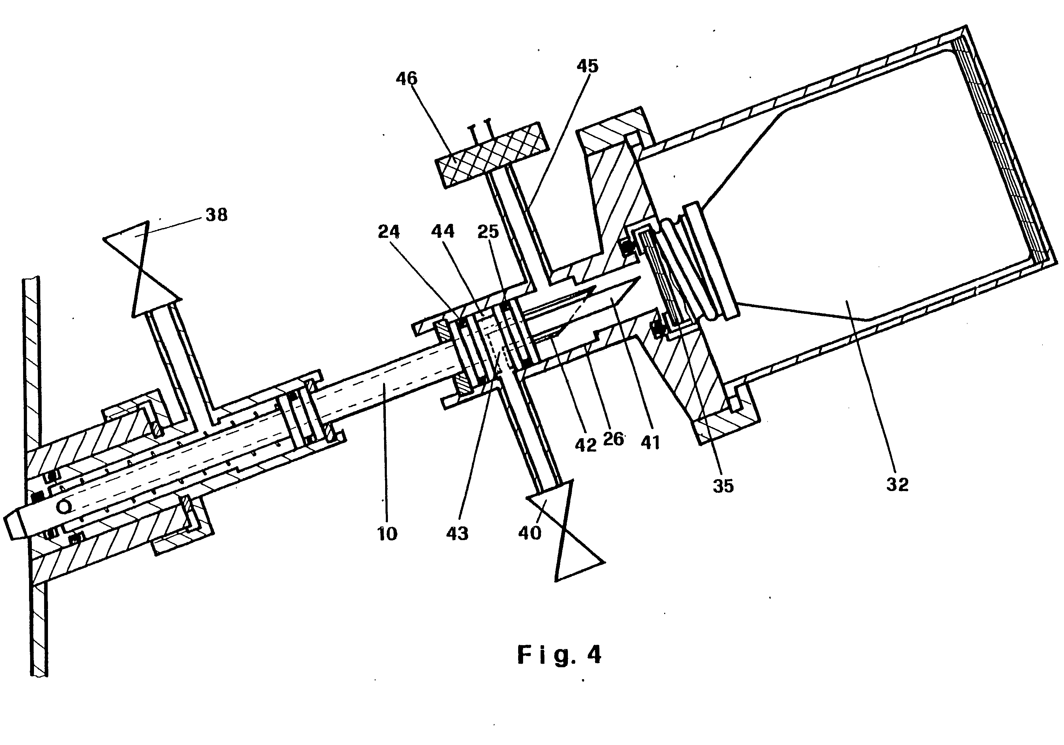

Der Einsatz der erfindungsgemässen Vorrichtung in Bio-Reaktoren oder Fermentern ist eine unter mehreren Einsatzmöglichkeiten. Sie ist ebenso geeignet, aus Chemie-Reaktoren mit flüssigem oder gasförmigem Inhalt Proben zu entnehmen, wie auch aus Lagertanks mit chemisch oder/und bakteriologisch instabilem Inhalt. Sollen bei drucklosen Reaktoren Proben gezogen werden in Flaschen ohne Unterdruck, so ist Entlüftung der Flasche 32 notwendig. In Fig. 4 ist eine solche erfindungsgemässe Variante als zweites Ausführungsbeispiel des Erfindungsgedankens dargestellt.

Hier sind anstelle der Nadel 36 zwei konzentrisch angeordnete Nadeln 41,42 vorhanden. Die innere Nadel 41 steht über eine Bohrung 43 in Verbindung mit einer zwischen den O-Ringen 24 und 25 liegenden Nut 44. In der Stellung der Vorrichtung gemäss Fig. 4 — der Sterilisationsstellung — sind beispielsweise die Absperr-Organe 38 und 40 geöffnet, und Dämpf, der beim Absperr-Organ 38 eingelassen wird, strömt, wie bekannt, durch das Entnahmerohr 10 und durch die äussere Nadel 42 in das Volumen innerhalb der Bohrung 26. Durch die Nadel 41 strömt der Dampf zurück und gelangt durch die Bohrung 43 in das — gegenüber dem Ausführungsbeispiel gemäss Fig. 1 bis 3 gegen den Fermenter zu versetzte Rohr 29 und dann nach aussen.

In der (nicht gezeichneten) Arbeitsstellung I durchdringen nacheinander beide Nadeln 41,42 den Stopfen 35. Die Nut 44 liegt nun vor einem weiteren Rohr 45, das seinerseits in einen Filter 46 mündet, der geeignet ist, Mikroorganismen zurückzuhalten, Luft aber durchlässt.

In der Arbeitsstellung II, die wiederum Fig. 3 entnommen werden kann, dringt Kulturbrühe 2 durch die äussere Nadel 42 in die Flasche 32 ein, die daraus verdrängte Luft verlässt sie durch die Nadel 41, gelangt durch die Bohrung 43 in die Nut 44, von dort in's Rohr 45 und durch den Filter 46 ins Freie oder allenfalls in einen nicht gezeichneten Gassammler oder ein Messgerät (ebenfalls nicht gezeichnet).

Es ist ebenfalls im Erfindungsgedanken mitenthalten, die Anschlüsse der beiden Nadeln so zu vertauschen, dass die Nadel 41 mit dem Inneren des Rohrs 10 in direkter Verbindung steht und die Abluft durch die Nadel 42 geführt wird, die daher in die Bohrung 43 mündet.

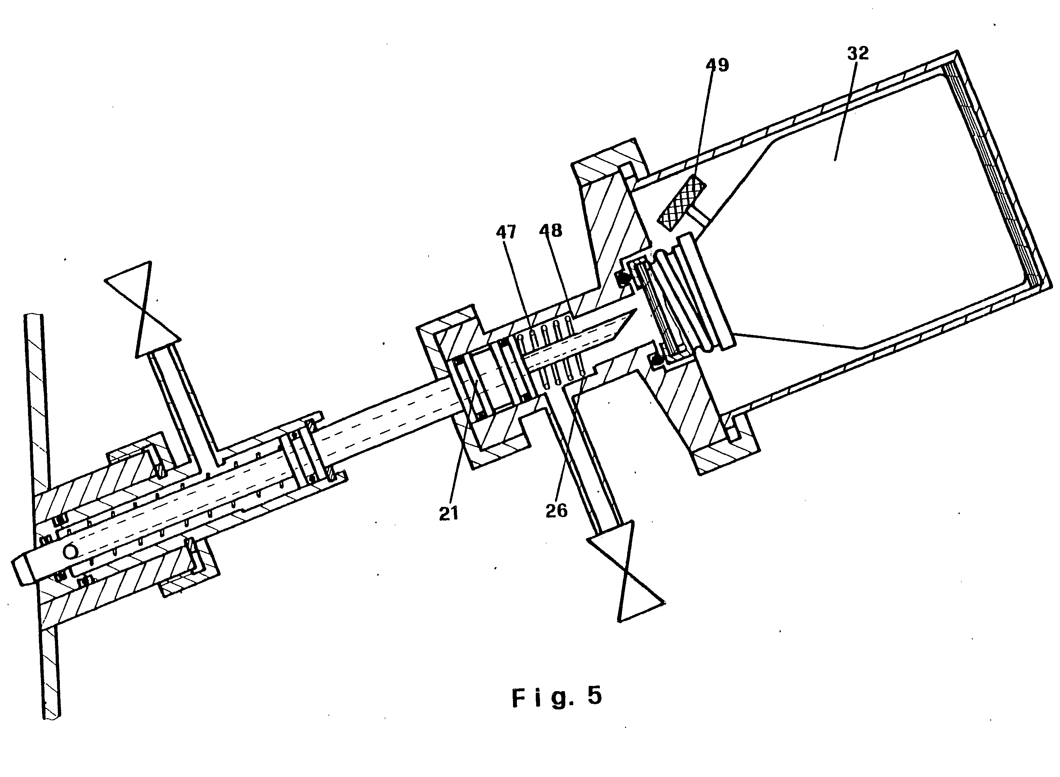

Fig. 5 zeigt eine weitere Variante des Ausführungsbeispiels gemäss Fig. 1. Die Variante besteht einerseits darin, dass eine zweite vorgespannte Druckfeder 47 vorhanden ist, die in die Bohrung 26 eingesetzt wird und einerseits gegen die Führung 21 und andererseits gegen eine Schulter 48 an der Bohrung 26 drückt. Diese Druckfeder 47 ist weicher eingestellt, als die Druckfeder 15, dergestalt dass sie vollständig komprimiert ist, bevor die Kompression der Druckfeder 15 beginnt. Die Variante gegenüber Fig. 1 besteht ferner in einem Filter 49, der direkt mit der Flasche 32 verbunden ist ; beispielsweise ist dies ein Glasfaser-Filter, mit den für den Filter 46 von Fig. 4 genannten Eigenschaften. Mit der Druckfeder 47 ist die ganze erfindungsgemässe Vorrichtung — nach Fig. 1 oder Fig. 4 oder Fig. 5 bedienbar dadurch, dass die in den Beschreibungen zu Fig. 1 bis 3 genannte axiale Kraft grösser oder kleiner gemacht wird ; eine Umkehrung der Kraftrichtung ist nicht erforderlich. Ein einfach wirkender Linearantrieb pneumatischer, hydraulischer oder elektrischer Natur kann diese Bewegung beispielsweise erbringen. Die Absperorgane 38,40 können beispielsweise Magnetventile sein. Wird anstelle der Ueberwurfmutter 30 am Flaschenhalter ein fernbedienbarer Schnellverschluss vorgesehen, so lässt sich der Probenahmebetrieb leicht automatisieren.

**Patentansprüche**

1. Vorrichtung zur Entnahme von Proben aus Behältern, wie Bio-Reaktoren, Fermentern, Chemie-Reaktoren oder Lagertanks, dadurch gekennzeichnet,

— dass sie besteht aus einem hohlen, mit dem Behälter fest zu verbindenden Hauptschaft (7) mit einem Rohr (9) zum Einleiten eines Sterilisationsmediums, einem mit einer Führung (16) im hohlen Hauptschaft (7) axial beweglichen Entnahmerohr (10) und einem mittels einer weiteren Führung (21) auf dem Entnahmerohr (10) axial beweglichen Flaschenhalter (27), in dessen Bohrung (26) die Führung (21) gleitet,

— dass das vorne gegen den Behälter zu verschlossene Entnahmerohr (10) eine seitliche Bohrung (18) aufweist, die in sein Inneres führt,

— dass das vom Behälter abgewandte Ende des Entnahmerohres (10) als eine mit einer Spitze (37) versehene Nadel (36) ausgebildet ist,

— dass in die Bohrung (26) des Flaschenhalters (27) die Mündung (39) mindestens eines weiteren Rohres (29) eingeführt ist zur Ableitung des beim Rohr (9) eingeleiteten Sterilisationsmediums,

— dass der Flaschenhalter (27) ausgebildet ist zur Befestigung eines Bechers (31), der die zur Aufnahme der Probe vorgesehene Flasche (32) birgt,

— dass ein erster O-Ring (14) mit dem Aeussern des Entnahmerohres (10) eine Dichtung bildet, die das Innere des Behälters gegen das Innere des Hauptschaftes (7) abdichtet, ein zweiter O-Ring (20) vorhanden ist, der in einer Nut (19) der Führung (16) liegt und das Innere des Hauptschaftes (7) gegen die Aussenwelt abdichtet, dass zwei weitere O-Ringe (24,25) in zwei Nuten (22,23) der Führung (21) liegen und die Aussenwelt gegen das Innere des Flaschenhalters (27) abdichten, und dass ein O-Ring (33) vorhanden ist, der, zwischen Flaschenhalter (27) und Schraubdeckel (34) der Flasche (32) gepresst, ebenfalls das Innere des Flaschenhalters (27) gegen die Aussenwelt abdichtet,

— dass die axiale Beweglichkeit der mit dem Entnahmerohr (10) fest verbundenen Führung (21) so bemessen ist, dass in der einen Extremstellung das aus der Nadel (36) ausströmende Sterilisationsmedium den ganzen Innenraum des Flaschenhalters (27) erreicht und ihn durch das Rohr (29) wieder verlässt, in der anderen Extremstellung die Führung (21) die Mündung (39) des Rohres (29 verschliesst, und die Nadel (36) durch den Pfropfen (35) der Flasche (32) hindurchgetreten ist, und

— dass die axiale Beweglichkeit des Entnahmerohres (10) im Hauptschaft (7) so bemessen ist, dass es in seiner einen Extremstellung so weit in den Behälter hineinragt, dass die Bohrung (18) in eine Kulturbrühe (2) eintaucht, in der anderen Extremstellung völlig innerhalb des hohlen Hauptschaftes (7) sich befindet.

2. Probenahme-Vorrichtung nach Patentanspruch 1, dadurch gekennzeichnet,

dass sich im Inneren des hohlen Hauptschaftes (7) eine zu diesem und zum Entnahmerohr (10) koaxiale, vorgespannte Druckfeder (15) befindet, die sich auf der dem Behälter zugewendeten Seite gegen den Hauptschaft (7) auf der anderen Seite gegen die mit dem Entnahmerohr (10) fest verbundene Führung (16) abstützt, und so dimensioniert ist, dass sie das Entnahmerohr (10) bei Wegfall einer äusseren, axial wirkenden Kraft selbsttätig aus dem Behälter zurückzieht.

3. Probenahme-Vorrichtung nach Patentanspruch 2, dadurch gekennzeichnet,

dass sich im Inneren des Flaschenhalters (27) eine zu diesem und zur Nadel (36) koaxiale, vorgespannte Druckfeder (47) befindet, die sich auf der einen Seite gegen eine Schulter (48) im Inneren des Flaschenhalters (27), auf der anderen Seite gegen die Führung (21) abstützt und so dimensioniert ist, dass die axiale Kraft, die zu ihrer Kompression aufzubringen ist, kleiner bleibt als die Kraft, die bei der Druckfeder (15) die Kompression einleitet.

4. Probenahme-Vorrichtung nach einem der Patentansprüche 1, 2 oder 3, dadurch gekennzeichnet,

— dass anstelle der Nadel (36) zwei koaxiale Nadeln (41,42) vorhanden sind, wobei die eine der Nadeln (41,42) die Fortsetzung des Entnahmerohres (10) darstellt, die andere der Nadeln (41,42) durch eine Bohrung (43) mit einer in der Führung (21) zwischen den O-Ringen (24,25) liegenden Nut (44) verbunden ist,

— dass ein weiteres Rohr (45) in die Bohrung (26) mündet, an welches sich ein Filter (46) anschliesst,

— dass die axiale Beweglichkeit der Führung (21) so bemessen ist, dass in der einen Extremstellung das aus einer der Nadeln (41,42) ausströmende Sterilisationsmedium den ganzen Innenraum des Flaschenhalters (27) erreicht und ihn durch die andere der Nadeln (41,42), die Bohrung (43), die Nut (44) und das Rohr (29) wieder verlässt, in der anderen Extremstellung die Nadeln (41,42) durch den Pfropfen (35) der Flasche (32) hindurchgetreten sind, und die in der Flasche (32) enthaltene Luft durch diejenige der Nadeln (41,42), die die Fortsetzung des Entnahmerohres (10) darstellt, weiter durch die Bohrung (43), die Nut (44), das Rohr (45) und den Filter (46) nach aussen strömt.

5. Probenahme-Vorrichtung nach Patentanspruch 4, dadurch gekennzeichnet,

dass die innere Nadel (41) die Fortsetzung des Entnahmerohres (10) darstellt.

6. Probenahme-Vorrichtung nach Patentanspruch 4, dadurch gekennzeichnet,

dass die äussere Nadel (42) die Fortsetzung des Entnahmerohres (10) darstellt.

7. Probenahme-Vorrichtung nach Patentanspruch 5 oder 6, dadurch gekennzeichnet, dass die innere Nadel (41) länger ist als die äussere Nadel (42).

8. Probenahme-Vorrichtung nach einem der Patentansprüche 1, 2 oder 3, dadurch gekennzeichnet,

dass die Flasche (32) mit einem Filter (49) versehen ist für die aus ihr verdrängte Luft.

## Claims

1. Device for the removal of samples from containers, such as bio-reactors, fermenters, chemical reactors or storage tanks, characterized in that

— it consists of a hollow main shaft (7), to be firmly connected to the container, with a tube (9) for the introduction of a sterilization medium, a removal tube (10), which is axially movable with a guide (16) in the hollow main shaft (7), and a bottle holder (27), which is axially movable by means of a further guide (21) on the removal tube (10), in the bore (26) of which bottle holder the guide (21) slides,

— that the removal tube (10), which is sealed to the front towards the container, has a lateral bore (18), which leads into its interior,

— that the end of the removal tube (10) facing away from the container is constructed as a needle (36) provided with a point (37),

— that the orifice (39) of at least one further tube (29) is introduced into the bore (26) of the bottle holder (27) to draw off the sterilization medium introduced at the tube (9),

— that the bottle holder (27) is constructed for the attachment of a beaker (31), which houses the bottle (32) which is provided to receive the sample,

— that a first O-ring (14) forms a seal with the exterior of the removal tube (10), which seal seals the interior of the container with respect to the interior of the main shaft (7), a second O-ring (20) is present, which lies in a groove (19) of the guide (16) and seals the interior of the main shaft (7) with respect to the environment, that two further O-rings (24,25) lie in two grooves (22,23) of the guide (21) and seal the environment with respect to the interior of the bottle holder (27), and that an O-ring (33) is present which, pressed between the bottle holder (27) and the screw cover (34) of the bottle (32), likewise seals the interior of the bottle holder (27) with respect to the environment,

— that the axial mobility of the guide (21), which is firmly connected with the removal tube (10), is dimensioned such that in one extreme position the sterilization medium flowing out from the needle (36) reaches the entire interior of the bottle holder (27) and leaves it again through the tube (29), in the other extreme position the guide (21) closes the orifice (39) of the tube (29), and the needle (36) has penetrated through the plug (35) of the bottle (32) and

— that the axial mobility of the removal tube (10) in the main shaft (7) is dimensioned such that in its one extreme position it projects into the container so far that the bore (18) dips into a culture liquor (2), and in the other extreme position is situated entirely within the hollow main shaft (7).

2. Sampling device according to Patent Claim 1, characterized in that in the interior of the hollow main shaft (7) there is situated a prestresed pressure spring (15), coaxial thereto and to the removal tube (10), which spring rests on the side facing the container against the main shaft (7), and on the other side against the guide (16), which is firmly connected with the removal tube (10), and which spring is dimensioned such that on elimination of an external, axially acting force, it automatically draws the removal tube (10) back out of the container.

3. Sampling device according to Patent Claim 2, characterized in that in the interior of the bottle holder (27) there is situated a prestressed pressure spring (47), which is coaxial thereto and to the needle (36), which rests on one side against a shoulder (48) in the interior of the bottle holder (27), and on the other side against the guide (21) and is dimensioned such that the axial force, which is to be applied for its compression, remains lower than the force which initiates the compression of the pressure spring (15).

4. Sampling device according to one of Patent Claims 1,2 or 3, characterized in that
— in place of the needle (36) two coaxial needles (41,42) are present, in which one of the needles (41,42) represents the continuation of the removal tube (10), the other of the needles (41,42) is connected through a bore (43) with a groove (44) lying in the guide (21) between the O-rings (24,25),
— that a further tube (45) opens into the bore (26), to which a filter (46) is adjacent,
— that the axial mobility of the guide (21) is dimensioned such that in one extreme position the sterilization medium flowing out of one of the needles (41,42) reaches the entire interior of the bottle holder (27) and leaves it again through the other of the needles (41,42), the bore (43), the groove (44) and the tube (29), in the other extreme position the needles (41,42) have penetrated through the plug (35) of the bottle (32), and the air contained in the bottle (32) flows through the needle of needles (41,42) which represents the continuation of the removal tube (10), on through the bore (43), the groove (44), the tube (45) and the filter (46) to the exterior.

5. Sampling device according to Patent Claim 4, characterized in that the inner needle (41) represents the continuation of the removal tube (10).

6. Sampling device according to Patent Claim 4, characterized in that the outer needle (42) represents the continuation of the removal tube (10).

7. Sampling device according to Patent Claim 5 or 6, characterized in that the inner needle (41) is longer than the outer needle (42).

8. Sampling device according to one of Patent Claims 1, 2 or 3, characterized in that the bottle (32) is provided with a filter (49) for the air displaced therefrom.

## Revendications

1. Dispositif pour prélever des échantillons dans des récipients tels que des réacteurs biologiques, fermenteurs, réacteurs chimiques ou réservoirs de stockage, caractérisé

en ce qu'il comprend un fût principal creux (7), qui est destiné à être fixé rigidement au récipient, un tube (9) servant à introduire un agent de stérilisation, un tube de prélèvement (10) qui peut se déplacer en translation axiale dans le fût principal creux (7) à l'aide d'un guide (16), et un support de flacon (27) que l'on peut déplacer en translation axiale sur le tube de prélèvement (10) au moyen d'un autre guide (21), et dans l'alésage (26) duquel le guide (21) coulisse,

en ce que le tube de prélèvement (10) qui est fermé à l'avant, vers le récipient, présente un perçage latéral (18) qui débouche dans son volume intérieur,

en ce que l'extrémité du tube de prélèvement (10) qui est à l'opposé du récipient est conformée en aiguille (36) munie d'une pointe (37),

en ce que, dans l'alésage (26) du support de flacon (27), débouche l'orifice (39) d'au moins un autre tube (29), qui sert à évacuer l'agent de stérilisation introduit par le tube (9),

en ce que le support de flacon (27) est constitué pour permettre d'y fixer un bécher (31) qui renferme le flacon (32) prévu pour recevoir l'échantillon,

en ce qu'une première bague torique (14) forme, avec la surface externe du tube de prélèvement (10), un joint étanche qui isole hermétiquement le volume intérieur du récipient du volume intérieur du fût principal (7), qu'il est prévu une deuxième bague torique (20) qui se trouve dans une gorge (19) du guide (16) et qui isole hermétiquement le volume intérieur du fût principal (7) de l'environnement extérieur,

en ce que deux autres bagues toriques (24, 25) se trouvent dans deux gorges (22, 23) du guide (21) et isolent hermétiquement l'environnement extérieur du volume intérieur du support de flacon (27) et qu'il est prévu une bague torique (33) qui, étant comprimée entre le support de flacon (27) et le bouchon vissable (34) du flacon (32), isole également hermétiquement le volume intérieur du support de flacon (27) de l'environnement extérieur,

en ce que la liberté de mouvement axial du guide (21) fixé rigidement au tube de prélèvement (10) est calculée de manière que, dans l'une de ses positions extrêmes, l'agent de stérilisation sortant de l'aiguille (36) atteigne la totalité du volume intérieur du support de flacon (27) et en ressorte par le tube (29) tandis que, dans l'autre position extrême, le guide (21) ferme l'orifice (39) du tube (29) et l'aiguille (36) est enfoncée à travers l'opercule (35) du flacon (32), et

en ce que la liberté de mouvement axial du tube de prélèvement (10) dans le fût principal (7) est calculée de manière que, dans l'une de ses positions extrêmes, ce tube pénètre suffisamment loin dans le récipient pour que le perçage (18) plonge dans le bouillon de culture (2) tandis que, dans l'autre position extrême, le perçage se trouve entièrement à l'intérieur du fût principal creux (7).

2. Dispositif de prélèvement d'échantillons selon la revendication 1, caractérisé

en ce qu'à l'intérieur du fût principal creux (7), se trouve un ressort de compression précontraint (15) coaxial à ce fût et au tube de prélèvement (10), qui prend appui, sur le côté dirigé vers le récipient, contre le fût principal (7) et, de l'autre côté, contre le guide (16) fixé rigidement au tube de prélèvement (10), et qui est calculé de manière à rétracter automatiquement le tube de prélèvement (10) du récipient en l'absence d'une force extérieure agissant dans la direction axiale.

3. Dispositif de prélèvement d'échantillons selon la revendication 2, caractérisé

en ce qu'à l'intérieur du support de flacon (27) se trouve un ressort de compression précontraint (47) coaxial à ce support et à l'aiguille (36) qui prend appui, d'un côté, contre un épaulement (48) formé à l'intérieur du support de flacon (27) et, de l'autre côté, contre le guide (21) et qui est calculé de telle manière que la force axiale que l'on doit développer pour le comprimer reste plus petite que la force qui détermine la compression du ressort de compression (15).

4. Dispositif de prélèvement d'échantillons selon l'une des revendications 1, 2 et 3, caractérisé

en ce qu'en remplacement de l'aiguille (36), il est prévu deux aiguilles coaxiales (41, 42), l'une des aiguilles (41, 42) représentant le prolongement du tube de prélèvement (10) tandis que l'autre des aiguilles (41, 42) est reliée par un perçage (43) à une gorge (44) située dans le guide (21) entre les bagues toriques (24, 25),

en ce qu'un autre tube (45) auquel se raccorde un filtre (46) débouche dans l'alésage (26),

en ce que la liberté de mouvement axial du guide (21) est calculée de manière que, dans une des positions extrêmes, l'agent de stérilisation qui sort de l'une des aiguilles (41, 42) atteigne la totalité du volume intérieur du support de flacon (27) et en ressorte par l'autre des aiguilles (41, 42), le perçage (43), la gorge (44) et le tube (29) tandis que, dans l'autre position extrême, les aiguilles (41, 42) sont enfoncées à travers l'opercule (35) du flacon (32) et l'air contenu dans le flacon (32) s'échappe à l'extérieur par celle des aiguilles (41, 42) qui représente le prolongement du tube de prélèvement (10), puis par le perçage (43), la gorge (44), le tube (45) et le filtre (46).

5. Dispositif de prélèvement d'échantillons selon la revendication 4, caractérisé

en ce que l'aiguille intérieure (41) représente le prolongement du tube de prélèvement (10).

6. Dispositif de prélèvement d'échantillons selon la revendication 4, caractérisé

en ce que l'aiguille extérieure (42) représente le prolongement du tube de prélèvement (10).

7. Dispositif de prélèvement d'échantillons selon la revendication 5 ou 6, caractérisé

en ce que l'aiguille intérieure (41) est plus longue que l'aiguille extérieure (42).

8. Dispositif de prélèvement d'échantillons selon l'une des revendications 1, 2 et 3, caractérisé

en ce que le flacon (32) est muni d'un filtre (49) pour l'air qui en est expulsé.

Fig. 1

0 172 838

Fig. 2

0 172 838

**Fig. 3**

**Fig. 4**

0 172 838

0 172 838

Fig. 5